(19) Europäisches Patentamt
European Patent Office
Office européen des brevets

(11) Veröffentlichungsnummer: **0 000 013**
**B1**

(12) # EUROPÄISCHE PATENTSCHRIFT

(21) Anmeldenummer: **78100025.2**

(22) Anmeldetag: **01.06.78**

(51) Int. Cl.³: **C 07 D 217/04,**
**A 61 K 31/47**

(54) **4-Phenyl-8-amino-tetrahydroisochinoline, diese enthaltende pharmazeutische Präparate und Verfahren zur Herstellung dieser Präparate**

(30) Priorität: **01.06.77 DE 2724610**

(43) Veröffentlichungstag der Anmeldung:
**20.12.78 Patentblatt 78/01**

(45) Bekanntmachung des Hinweises auf die Patenterteilung:
**15.10.80 Patentblatt 80/21**

(84) Benannte Vertragsstaaten:
**BE CH DE FR GB NL SE**

(56) Entgegenhaltungen:
**Keine**

(73) Patentinhaber: **HOECHST AKTIENGESELLSCHAFT**
**Postfach 80 03 20 D - 6230 Frankfurt**
**Main 80 (DE)**

(72) Erfinder: **Schmitt, Karl, Dr.**
**Hasenpfad 3**
**D - 6232 Bad Soden am Taunus (DE)**
**Hoffmann, Irmgard, Dr.**
**Oranienstrasse 1**
**D - 6232 Bad Soden am Taunus (DE)**
**Schacht, Ulrich, Dr.**
**Finkenweg 12**
**D - 6238 Hofheim am Taunus (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

EP 0 000 013 B1

# 4-Phenyl-8-amino-tetrahydroisochinoline, diese enthaltende pharmazeutische Präparate und Verfahren zur Herstellung dieser Präparate

Aus der deutschen Patentschrift 1 795 829 sind 4-Phenyl-8-amino-tetrahydroisochinoline der allgemeinen Formel I

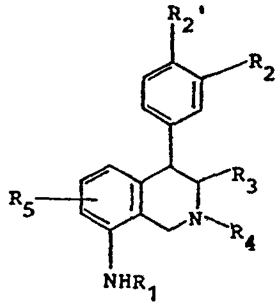

I

worin $R_1$ Wasserstoff, eine niedere Alkyl- oder die Benzylgruppe, $R_2$ Wasserstoff, die Methylgruppe, Chlor- oder Fluoratome, $R_2'$ Wasserstoff, Methyl-, Methoxy-, Hydroxygruppen oder Halogenatome, $R_3$ und $R_4$ Wasserstoff oder eine niedere Alkylgruppe und $R_5$ Wasserstoff, ein Chloratom oder eine Methoxygruppe in 5- oder 6-Stellung bedeuten, bekannt.

Es wurde nun überraschenderweise gefunden, daß zwei bisher nicht bekannte Verbindungen die vorbekannte Verbindungsklasse der obigen Formel I in ihrer antidepressiven Wirkung erheblich übertreffen.

Gegenstand der Auswahlerfindung sind somit Verbindungen der allgemeinen Formel II

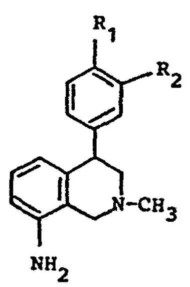

II

in der entweder $R_1$ für Brom und $R_2$ für Wasserstoff stehen oder sowohl $R_1$ als auch $R_2$ Chlor bedeuten.

Die neuen Verbindungen der allgemeinen Formel II Zeichnen sich u.a. durch eine gegenüber der vorbekannten Verbindungsklasse verstärkte Wirkung im Serotonin-Aufnahme-Hemmtest aus. Während die vorbekannten Verbindungen der Formel I (mit Ausnahme von Verbindungen der Formel II) erst in höherer Konzentration die Serotonin-Aufnahme hemmen, bewirken die Verbindungen der Formel II eine Serotonin-Aufnahme-Hemmung, wie man sie bisher mit den Verbindungen der Formel I nicht erzielen konnte.

Die Serotonin-Aufnahme-Hemmung äußert sich in einer verstärkten seelischen stimmungsaufhellenden Wirkung und hat daher für die Therapie erhebliche Bedeutung. Die Verbindungen der Formel II sind daher wertvolle Pharmazeutika, die zur Behandlung von depressiven Zuständen verwendet werden.

Die Herstellung der Verbindungen der Formel II erfolgt nach dem Verfahren der deutschen Patentschrift 1 670 694 durch Cyclisierung der entsprechenden $\alpha$-[N-(2-Aminobenzyl)-N-methylaminomethyl]-benzylalkohole.

Die Verbindungen der Formel II können sowohl mit einem als auch mit zwei Äquivalenten einer Säure Salze bilden. Im Hinblick auf ihre Verwendung als Heilmittel kommen für die Salzbildung physiologisch verträgliche Säuren in Betracht. Als anorganische Säuren kommen beispielsweise in Betracht: Halogenwasserstoffsäuren, wie Chlorwasserstoffsäure und Bromwasserstoffsäure sowie Schwefelsäure, Phosphorsäure und Amidosulfonsäure. Als organische Säuren seien beispielsweise genannt: Ameisensäure, Essigsäure, Propionsäure, Milchsäure, Glykolsäure, Gluconsäure, Maleinsäure, Bernsteinsäure, Weinsäure, Benzoesäure, Salicylsäure, Zitronensäure, Acetursäure oder Oxyäthansulfonsäure.

## Beispiel 1

50 g 3,4-Dichloracetophenon werden in 300 ml methylenchlorid mit Brom bei Raumtemperatur bromiert. Das so erhaltene $\omega$-Brom-3,4-dichloracetophenon wird direkt verwendet. Zunächst wird die $\omega$-Bromverbindung in 300 ml Äthanol gelöst. Unter Rühren läßt man bei 60°C die Lösung von 42,6 g N-Methyl-2-nitrobenzylamin und 33,1 g N - Äthyl - N,N - diisopropyläthylamin in 100 ml Äthanol hinzutropfen. Nun wird noch 1 Stunde bei Raumtemperatur, dann 2 Stunden unter Sieden gerührt, darauf dampft man zur Trockne ein. Den Rückstand löst man in Wasser und Äther; der letztere wird abgetrennt, mit Kaliumcarbonat gretrocknet und abermals eingedampft.

Der Rückstand bildet mit Athanol/Chlorwasserstoff ein Salz, und man erhält 19 g N - (3,4 - Dichlorphenacyl) - N - methyl - 2 - nitro - benzylamin - hydrochlorid, Fp 165°C (Zers.).

Aus dem Hydrochlorid stellt man wieder die Base her, die mit Methylenchlorid als Öl (18 g) isoliert wird. Carbonylund Nitrogruppe werden nun nacheinander hydriert. Die Carbonylgruppe gibt mit Natrium-borhydrid (5 g in 50 ml Methanol zu 18 g Acetophenonderivat in 250 ml Methanol) 15 g der Hydroxyverbindung, die Nitrogruppe hydriert man nun mit Raney-Nickel bei Normaldruck und Zimmertemperatur. Die Wasserstoffaufnahme ist wie berechnet, man erhält 14 g $\alpha$ - [N - 2 - Aminobenzyl - N - methylamino - methyl] - 3,4 - dichlorbenzylalkohol als öliges Produkt.

Zur Cyclisierung werden 14 g des erhaltenen Öls in 100 ml Methylenchlorid unter Rühren bei 5 bis 10°C in 70 ml Schwefelsäure (conz.), eingetropft. Man rührt eine Stunde bei Raumtemperatur nach und gießt schließlich auf zerstroßenes Eis. Unter weiterer Kühlung wird mit conz. NaOH neutralisiert, wobei das Reaktionsprodukt in öliger Form ausfällt. Man isoliert die Base mit Methylenchlorid und erhält 8 g des Isochinolinderivates als Base.

Die Base wird mit Maleinsäure (3 g für 8 g Base) in Äthanol unter Erwärmen gelöst. Das beim Abkühlen kristallisierende Maleinat wird aus Äthanol umkristallisiert.

Man erhält 3,5 g 8 - Amino - 4 - (3,4 - dichlorphenyl) - 2 - methyl - 1,2,3,4 - tetrahydroisochinolin - hydrogenmaleinat vom Schmelzpunkt Fp 180—183°C.

Beispiel 2

Ausgehend von 4-Bromacetophenon erhält man gemäß der in Beispiel 1 beschriebenen Arbeitsweise das 8-(Amino-4-(4-bromophenyl)-2 - methyl - 1,2,3,4 - tetrahydroisochinolinhydrogenmaleinat, das bei 189—191°C schmilzt.

**Patentansprüche:**

1. 4 - Phenyl - 8 - amino - tetrahydroisochinoline der Formel

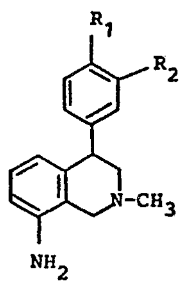

in der entweder $R_1$ für Brom und $R_2$ für Wasserstoff stehen oder sowohl $R_1$ als auch $R_2$ Chlor bedeuten und deren Salze mit physiologisch unbedenklichen Säuren.

2. Pharmazeutische Präparate mit antidepressiver Wirkung gekennzeichnet durch einen Gehalt an Verbindungen nach Anspruch 1.

3. Verfahren zur Herstellung von pharamzeutischen Präparaten mit antidepressiver Wirkung, dadurch gekennzeichnet, daß man eine Verbindung nach Anspruch 1, gegebenenfalls mit üblichen Trägerstoffen und/oder Stabilisatoren in eine pharmazeutisch verträgliche Anwendungsform bringt.

**Claims**

1. 4 - Phenyl - 8 - amino - tetrahydroisoquinolines of the formula

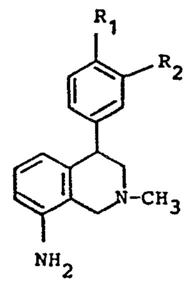

in which either $R_1$ denotes bromine and $R_2$ denotes hydrogen or $R_1$ as well as $R_2$ denote chlorine, and their salts with physiologically tolerated acids.

2. Pharmaceutical compositions having antidepressive action characterized by a content of a compound as claimed in claim 1.

3. Process for the manufacture of pharmaceutical compositions having antidepressive action, characterized in that a compound as claimed in claim 1, optionally together with usual carriers and/or stabilizers, are brought into a form suitable for therapeutical administration.

**Revendications**

1. 4 - Phényl - 8 - amino - tétrahydroisoquinoléines de formule:

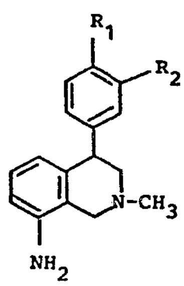

dans laquelle $R_1$ représente un atome de brome et $R_2$ un atome d'hydrogène ou $R_1$ et $R_2$ représentent chacun un atome de chlore, et leurs sels avec des acides physiologiquement acceptables.

2. Compositions pharmaceutiques ayant un effet antidépresseur, caractérisées en ce qu'elles contiennent un composé selon la revendication 1.

3. Procédé de préparation de compositions pharmaceutiques ayant un effet antidépresseur, cáractérisé en ce qu'on met sous une forme pharmaceutiquement acceptable un composé selon la revendication 1, éventuellement avec des véhicules et/ou des stabilisants.